# EUROPEAN PATENT SPECIFICATION

(11) **EP 0 710 098 B1**
(45) Date of publication and mention of the grant of the patent: **30.12.1998**
(21) Application number: 94922675.7
(22) Date of filing: 22.07.1994
(51) Int. Cl.: A61F 13/15

(54) **ABSORBENT ARTICLES HAVING UNDERGARMENT COVERING COMPONENTS PRE-DISPOSED TO AUTOMATICALLY WRAP THE SIDES OF UNDERGARMENTS**
SAUGFÄHIGE ARTIKEL MIT BESTANDTEILEN DIE AUTOMATISCH DIE SEITEN VON UNTERWÄSCHE ZU DEREN SCHUTZ UMWICKELN
ARTICLES ABSORBANTS POSSEDANT DES ELEMENTS DE COUVERTURE DES SOUS-VETEMENTS PREDISPOSES POUR ENVELOPPER AUTOMATIQUEMENT LES COTES DES SOUS-VETEMENTS

(30) Priority: 22.07.1993 US 96121; 20.09.1993 US 124180
(43) Date of publication of application: 08.05.1996
(73) Proprietor: THE PROCTER & GAMBLE COMPANY, Cincinnati, Ohio 45202 (US)
(72) Inventor: OSBORN, Thomas, Ward, III, Cincinnati, OH 45224 (US)
(74) Representative: Hirsch, Uwe Thomas
(86) International application number: US9408256
(87) International publication number: WO9503024

(56) References cited:
- EP-A- 0 331 018
- EP-A- 0 337 438
- EP-A- 0 467 184
- EP-A- 0 511 905
- WO-A-93/12747

## Description

### FIELD OF THE INVENTION

The present invention relates to absorbent articles such as sanitary napkins, panty liners, and incontinence pads. More particularly, the present invention relates to sanitary napkins that have undergarment covering components (or "side wrapping elements") that are pre-disposed to automatically wrap the sides of a wearer's undergarments when the undergarments are pulled up to provide an alternative to conventional side flaps.

### BACKGROUND OF THE INVENTION

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perineal area of the body. Sanitary napkins both with and without side flaps (or wings) are disclosed in the literature and are available in the marketplace.

Generally when sanitary napkins are provided with flaps, the flaps extend laterally from a central absorbent means and are intended to be folded around the edges of the wearer's panties in the crotch region. Commonly, the flaps are provided with an attachment means for either affixing the flaps to the underside of the wearer's panties or to the opposing flap. The flaps are particularly effective for preventing exudates from soiling the edges of the wearer's panties.

Sanitary napkins having flaps of various types are disclosed in U.S. Patent 4,687,478, entitled "Shaped Sanitary Napkin With Flaps", which issued to Van Tilburg on August 18, 1987; U.S. Patent 4,608,047, entitled "Sanitary Napkin Attachment Means", which issued to Mattingly on August 26, 1986; U.S. Patent 4,589,876, entitled "Sanitary Napkin", which issued to Van Tilburg on May 20, 1986 and its Reexamination Patent No. B1 4,589,876, Certificate of Reexamination issued April 27, 1993; U.S. Patent 4,285,343, entitled "Sanitary Napkin", which issued to McNair on August 25, 1981; U.S. Patent 3,397,697, entitled "Disposable Sanitary Shield For Undergarments", which issued to Rickard on August 20, 1968; and, U.S. Patent 2,787,271, entitled "Sanitary Napkin", which issued to Clark on April 2, 1957.

US-A-4,940,426 which is equivalent to EP-A-331018 discloses sanitary napkins having side flaps which are tensioned in longitudinal direction, typically by the addition of elastics, without providing automatic wrapping. EP-A-337438 discloses a sanitary napkin having biased hinges to connect side flaps and main body of the napkin. The flaps have to be unfolded prior to application of the napkin.

While sanitary napkins having flaps are commonly viewed as providing better protection against soiling as compared to sanitary napkins without flaps, some women find applying sanitary napkins having flaps to be inconvenient for various reasons. For instance, some women find it to be difficult to attach the flaps to the underside of the crotch of their panties. This can be due to factors such as the tendency for the adhesive fasteners on the flaps to stick to themselves or to other parts of the sanitary napkin. As a result, some women still prefer a sanitary napkin without flaps. In addition, some women who generally prefer a sanitary napkin with flaps, occasionally (such as during periods of light flow) prefer a sanitary napkin without flaps. Therefore, there is a need for a sanitary napkin which provides an alternative to sanitary napkins having conventional side flaps while still providing the protection of side flaps.

Several variations of sanitary napkins having conventional flaps that attempt to solve some, but not all of these problems are disclosed in the patent literature. For example, U.S. Patent 4,911,701 issued to Mavinkurve discloses a sanitary napkin having elastic strands for providing a greater convex shape to the body-facing portion of the central absorbent and for enabling adhesive-free placement of the flaps of a winged napkin embodiment into a pair of panties. The sanitary napkin described in the Mavinkurve patent, however, still appears to require the user to manipulate the flaps (by first flipping the flaps upward and then placing the flaps in her panties and flipping the flaps back down) since the flaps appear to be pre-disposed to be in a downward folded condition. The Mavinkurve patent also requires that individual elastic strands be attached in a contracted condition to the central absorbent portion of the napkin and/or to its wings or flaps. The napkins described in the Mavinkurve patent can, therefore, be difficult and expensive to manufacture. U.S. Patent 4,940,462 issued to Salerno discloses a sanitary napkin with longitudinally expandable flaps. The flaps are designed to fold over the exterior of the wearer's panty and then to longitudinally expand to conform with the contour of the panties. The Salerno patent, however, appears to require conventional adhesive fasteners to retain the flaps in place on the underside of the wearer's panties.

Thus, a need exists for an absorbent article, such as a sanitary napkin, that is provided with an alternative to conventional flaps. In particular, a need exists for a sanitary napkin having an alternative to conventional flaps which provides the protection from soiling of conventional flaps and which can conveniently and efficiently solve the problems caused when attempting to attach conventional flaps to the underside of the wearer's panties.

It is, therefore, an object of the present invention to provide an absorbent article, such as a sanitary napkin, that is able to provide coverage to the wearer's panties to reduce side soiling (i.e., staining of the edges of the panty crotch) without the use of conventional flaps.

It is another object of the present invention to provide an absorbent article, such as a sanitary napkin that automatically wraps around the sides of the wearer's panties by the simple action of the wearer pulling up her panties.

It is still another object of the present invention to provide an absorbent article, such as a sanitary napkin, that is able to wrap around the sides of the wearer's panties and stay without providing flaps having panty fasteners thereon, and without attaching separate elastic strands to the sanitary napkin.

These and other objects of the present invention will be more readily apparent when considered in reference to the following description and when taken in conjunction with the accompanying drawings.

### SUMMARY OF THE INVENTION

The present invention provides an absorbent article, such as a sanitary napkin. The sanitary napkin of the present invention has side wrapping elements that automatically wrap the sides of a wearer's undergarments when the undergarments are pulled up. The side wrapping elements provide an alternative to conventional side flaps which must be manually positioned into their in-use condition.

The sanitary napkin has a longitudinal dimension extending in a longitudinal direction and a transverse dimension extending in a transverse direction. The sanitary napkin comprises a main body portion and a pair of side wrapping elements. The main body portion comprises an absorbent core, and has a body-facing side, a garment-facing side, and a pair of longitudinal side edges. The side wrapping elements are joined to the main body portion and extend laterally outward beyond the longitudinal side edges of the main body portion from their proximal edges to their distal edges. At least one, and preferably both of the side wrapping elements are pre-disposed to automatically wrap the sides of the crotch region of the wearer's undergarments. The side wrapping elements, in one preferred embodiment, are pre-disposed to automatically wrap the sides of the wearer's undergarments by virtue of having end portions that are preferably placed under tension and folded back toward the longitudinal centerline of the sanitary napkin and joined to the garment-facing side of the main body portion.

The sanitary napkin of the present invention provides an alternative to sanitary napkins having conventional side flaps for several reasons. The side wrapping elements do not extend far enough outward beyond the side edges of the wearer's panties to cause any inconvenience to the wearer. The sanitary napkin requires no action on the part of the wearer in order to fold the side wrapping elements under their panties or to attach the same to their panties. The side wrapping elements, therefore, stay in place well enough to cover the sides edges of the wearer's panties without affixing them underneath the wearer's panties.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:
FIG. 1 is a schematic top plan view of the sanitary napkin of the present invention.
FIG. 2 is a schematic cross-sectional view of the sanitary napkin shown in FIG. 1 taken along line 2-2 of FIG. 1.
FIG. 3 is a schematic cross-sectional view of the sanitary napkin shown in FIG. 1 taken along lines 3-3 of FIG. 1.
FIG. 4 is a perspective view of the garment-facing side of the sanitary napkin shown in FIGS. 1-3.
FIG. 5 is a perspective view of the sanitary napkin shown in the preceding figures showing the folding of the side wrapping elements.

### DETAILED DESCRIPTION OF THE INVENTION

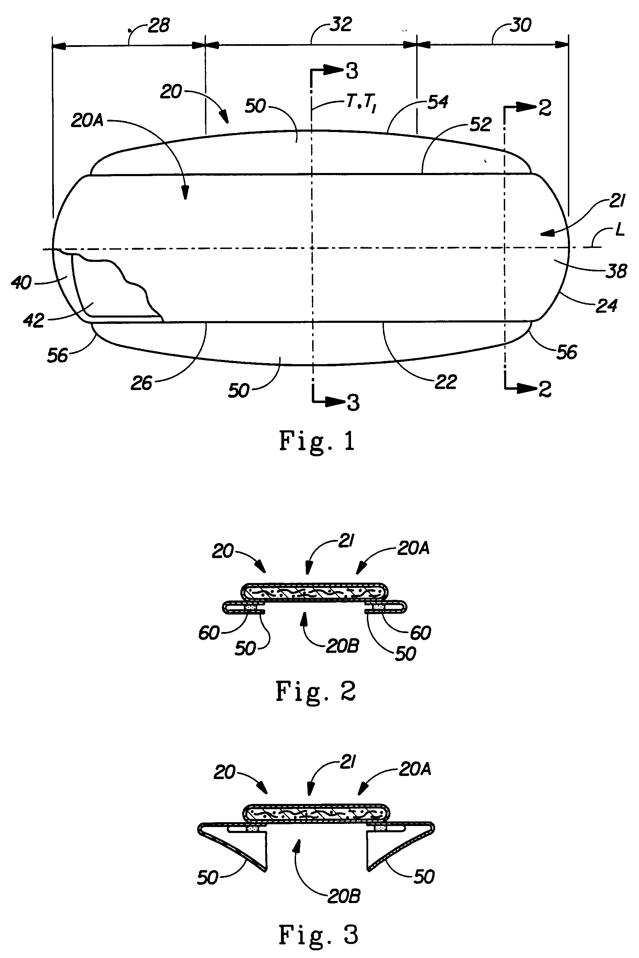

FIGS. 1-4 show one preferred embodiment of a disposable absorbent article of the present invention, sanitary napkin 20. More particularly, the present invention relates to sanitary napkins that have a main body portion 21 and a pair of side wrapping elements 50 that automatically wrap the sides of the wearer's panties when the wearer places the sanitary napkin in her panties and pulls her panties up.

The sanitary napkin 20 has two surfaces, a liquid pervious body-contacting surface or "body surface" 20A and a liquid impervious garment surface 20B. The sanitary napkin 20 is shown in FIG. 1 as viewed from its body surface 20A. The body surface 20A is intended to be worn adjacent to the body of the wearer. The garment surface 20B of the sanitary napkin 20 is intended to be placed adjacent to the wearer's undergarments when the sanitary napkin 20 is worn.

The sanitary napkin 20 has two centerlines, a longitudinal centerline L and a transverse centerline T. The term "longitudinal", as used herein, refers to a line, axis, or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis, or direction which lies within the plane of the sanitary napkin 20 that is generally perpendicular to the longitudinal direction.

FIG. 1 shows that the main body portion 21 of the sanitary napkin 20 comprises the portion of the sanitary napkin without the side wrapping elements. The main body portion 21 has two spaced apart longitudinal edges 22, two spaced apart transverse or end edges (or "ends") 24, which together form the periphery 26 of the main body portion of the sanitary napkin 20. The main body portion also has two end regions, which are designated first end region 28 and second end region 30. A central region 32 is disposed between the end regions 28 and 30. The end regions 28 and 30 extend outwardly from the edges of the central region 32 about 1/8 to about 1/3 of the length of the main body portion. A detailed description of a sanitary napkin having a central region 32 and the two end regions 28 and 30 is contained in U.S. Patent 4,690,680 issued to Higgins on September 1, 1987.

The main body portion 21 of the sanitary napkin 20 can be of any thickness, including relatively thick, relatively thin, or even very thin. The embodiment of the sanitary napkin 20 shown in Figures 1-4 of the drawings is intended to be an example of a relatively thin sanitary napkin, preferably an "ultra-thin" sanitary napkin as described in U.S. Patents 4,950,264 and 5,009,653, both issued to Osborn. The sanitary napkin 20 shown preferably should also be relatively flexible, so that it is comfortable for the wearer. It should be understood that the sanitary napkin shown is merely one preferred embodiment, and that the present invention is not limited to absorbent articles of the type or having the specific configurations shown in the drawings.

FIG. 1 shows the individual components of the main body portion 21 of the sanitary napkin 20 of the present invention. The main body portion 21 shown in FIG. 1 generally comprises at least three primary components. These include a liquid pervious topsheet 38, a liquid impervious backsheet 40, and an absorbent core 42 positioned between the topsheet 38 and the backsheet 40. There are occasions, however, when one or more of these components, such as the backsheet, can comprise part of the side wrapping elements described herein. The topsheet, the backsheet, and the absorbent core may be assembled in a variety of configurations known in the art (including so called "sandwich" products and "tube" products).

Several preferred sanitary napkin configurations are described generally in U.S. Patent 4,321,924, "Bordered Disposable Absorbent Article" issued to Ahr on March 30, 1982; U.S. Patent 4,425,130, "Compound Sanitary Napkin" issued to DesMarais on January 10, 1984; U.S. Patent 4,950,264, "Thin, Flexible Sanitary Napkin" issued to Osborn on August 21, 1990; U.S. Patent 5,308,346, "Elasticized Sanitary Napkin" issued to Sneller, et al. on May 3, 1994; PCT Patent Application WO 94/02096 entitled "Absorbent Articles Having Panty Covering Components That Naturally Wrap the Sides of Panties" having a priority of July 22, 1993, in the name of Lavash, et al.; and PCT Patent Application WO 95/07675 entitled "Absorbent Articles Having Panty Covering Components Comprising Extensible Web Materials Which Exhibit Elastic-Like Behavior" having a priority of September 17, 1993, in the name of Mansfield, et al. The main body portion 21 of the sanitary napkin may also be comprised of one or more extensible components, and preferably all extensible components such as those sanitary napkins, and the like described in PCT Publication Nos. WO 93/01785 and 93/01786, both published February 4, 1993.

Figure 1 shows a preferred embodiment of the sanitary napkin 20 assembled in a sandwich construction in which the topsheet 38 and the backsheet 40 have length and width dimensions generally larger than those of the absorbent core 42. The topsheet 38 and the backsheet 40 extend beyond the edges of the absorbent core 42 to thereby form portions of the periphery 26. The sanitary napkin 20 of the present invention comprises pair of side wrapping elements 50 joined to the main body portion 21 that extend laterally outward beyond the longitudinal side edges 22 of the main body portion 21 from proximal edges 52 to their distal edges 54. The side wrapping elements 50 can be joined to the main body portion 21 in any suitable manner. The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element.

The side wrapping elements 50 can be of any suitable size and shape. The side wrapping elements 50 can have a length (longitudinal dimension) that varies within a large range. The length of the side wrapping elements 50 is preferably at least as large as the longitudinal dimension of known types of flaps that attach to each other or to the underside of a wearer's panties. The side wrapping elements 50, therefore, can primarily extend only from the central region 32 of the main body portion of the sanitary napkin. Alternatively, the side wrapping elements 50 can have a length that is as long as, or longer than, the length of the main body portion 21. The distal edges 54 of the side wrapping elements preferably extend outward beyond the longitudinal side edges 22 of the main body portion 21, a distance of less than one-half the width of the main body portion. The side wrapping elements 50 of the present invention preferably have the dimensions set forth for the panty covering components in the aforementioned PCT Patent Application WO 94/02096 and WO 95/07675 filed in the names of Lavash, et al. and Mansfield, et al.,

The side wrapping elements 50 can be made from any of the materials suitable for use in the construction of the main body portion 21 of the sanitary napkin. These materials can be absorbent if the components of the side wrapping elements are suitably sealed to prevent the outward wicking of exudates from the distal edges of the side wrapping elements. Preferably, however, the components of the side wrapping elements are substantially non-absorbent. The components of the side wrapping elements that form the garment-facing side of the side wrapping elements should also preferably be liquid impervious. The materials comprising the side wrapping elements may either be inextensible, or extensible. If the side wrapping elements 50 are extensible, they can be extensible in the longitudinal direction, the transverse direction, in directions between the longitudinal and transverse directions, or in multi-directions.

In one preferred embodiment, the side wrapping elements comprise a laminate of three materials, a soft extensible coverstock material such as a longitudinally extensible spunbond nonwoven web, an extensible intermediate layer such as a three dimensional formed film, and an extensible liquid impervious backing such as a polyethylene film backsheet material. The coverstock material preferably comprises a spunbonded polyethylene nonwoven web such as Spunbond PE, which was obtained from Polybond, Incorporated of Waynesboro, VA or a product known as COROLIND PE, which was obtained from Corovin GMBH of Germany. The extensible intermediate layer preferably comprises a variation of a three dimensional formed film known as DRI-WEAVE which is used as a topsheet on sanitary napkins manufactured by the Procter & Gamble Company, Cincinnati, Ohio under U.S. Patents 4,342,314 issued to Radel, et al. and 4,463,045 issued to Ahr, et al. The three dimensional film has an embossed thickness of between (about 15 mils to about 35 mils) about 0.38 mm to about 0.89 mm and is not apertured all the way through as in the case of DRI-WEAVE topsheet material, but is formed so that the apertures are closed off on the side of the film that would ordinarily face away from the wearer's body in use. The formed film resin composition is modified by adding a blend of linear low density polyethylene ("LLDPE") and high density polyethylene ("HDPE") such that the formed film is capable of extending between about 60% and about 200% in the longitudinal direction. The formed film is preferably extrusion coated onto the nonwoven web. The polyethylene backsheet material is a film comprised of LLDPE that preferably has a caliper of between about 0.01016-0.0381 mm (about 0.4-1.5 mil) more preferably about 0.0254 mm (1 mil). A preferred polyethylene film is known as #P18-1401 and is obtained from Clopay Corporation of Cincinnati, Ohio. This polyethylene film can either be glued to the formed film side of the laminate of the nonwoven and formed film such as by a spiral application of adhesives, or it can be extruded thereon.

FIGS. 1-4, show that the side wrapping elements 50 are in a configuration that is pre-disposed to automatically wrap the sides of the wearer's undergarments. Specifically, the side wrapping elements are attached to the garment-facing side of the main body portion 21 so that they are biased to fold toward the garment-facing side of the main body portion. The side wrapping elements 50 each have a pair of ends 56 and a pair of end portions, such as distal end portions 58. The distal end portions 58 comprise portions of the side wrapping elements 50 that are adjacent to both the distal edges 54 of the side wrapping elements 50 and the end edges 56 of the side wrapping elements. In the preferred embodiment shown in the drawings, the distal end portions 58 are inflected (that is, folded back on the garment-facing side of the main body portion 21 toward the longitudinal centerline, L) and secured to the garment-facing side of the main body portion such as by adhesives 60. This pre-disposes the side wrapping elements 50 to fold toward the garment-facing side of the main body portion 21.

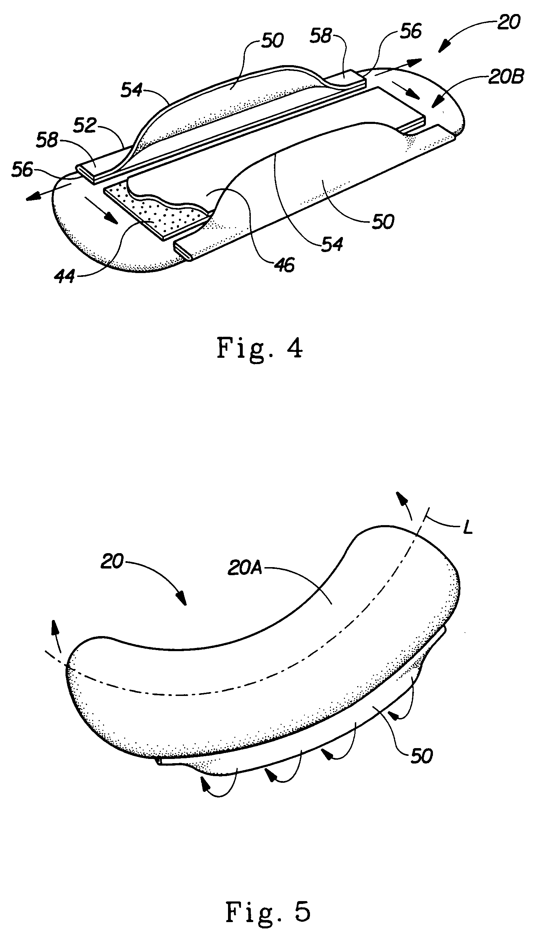

In particularly preferred embodiments, the side wrapping elements 50 are placed in tension when they are secured to the garment-facing side of the main body portion 21. The side wrapping elements can be placed in tension in the longitudinal direction, or in the transverse direction as shown by the arrows in FIG. 4, or in a direction between the longitudinal and transverse directions. This increases the tendency for the side wrapping elements 50 to wrap around the edge of the wearer's panties. The side wrapping elements 50 in these particularly preferred embodiments are preferably extensible so that they can be stretched more before they are secured to the main body portion 21.

The side wrapping elements 50 can be pre-disposed to form any suitable angle relative to the garment-facing side of the main body portion by varying the amount of tension on the distal edge portions 58 when they are secured to the main body portion. For instance, the side wrapping elements 50 can form an acute angle relative to the garment-facing side of the main body portion 21 as shown in FIG. 3. (The angles referred to herein are measured at a section along the transverse centerline T₁ of the side wrapping elements.) Alternatively, the side wrapping elements 50 can be pre-disposed at a 90° angle, or as shown in FIG. 4, at an obtuse angle relative to the garment-facing side of the main body portion 21. The latter arrangement may be somewhat preferred because it eliminates the need to flip the side wrapping elements 50 back outward before placing the sanitary napkin in the wearer's panties.

The side wrapping elements 50 preferably have a resistance to edge compression so that they will fold rather than crumple when placed in a wearer's panties and subjected to the forces associated with wearing the sanitary napkin. The term "resistance to edge compression" refers to how substantial the material that comprises the side wrapping elements 50 is. Specifically, edge compression refers to the tendency of the side wrapping elements 50 to buckle when the side wrapping elements are extended to form a planar extension and forces are applied to the distal edge 54 of the side wrapping element by a plate oriented perpendicular to the plane of the side wrapping elements 50. This property is important because if the side wrapping elements 50 are insubstantial, they will bunch up when forces are applied to the side wrapping elements by the wearer's panty elastics or by the wearer's thighs during wear. The side wrapping elements 50 should, of course, not be so stiff that they are uncomfortable to the wearer.

The garment surface 20B of the sanitary napkin 20 may include, and preferably does include, fasteners for attaching the sanitary napkin to the wearer's undergarments. Figure 4 shows the central pad fastener 44 which is adapted to secure the main body portion 21 of the sanitary napkin to the crotch region of an undergarment. Fasteners comprising adhesives have been found to work well for this purpose, with pressure-sensitive adhesives being preferred. Before the sanitary napkin 20 is placed in use, if an adhesive fastener is used, the adhesive is typically covered with a removable cover strip or release liner 46 in order to keep the adhesive from sticking to a surface other than the crotch portion of the panty prior to use. Suitable release liners are described in U.S. Patent 4,917,697.

The sanitary napkin 20 of the present invention is used by removing any release liner and thereafter placing the sanitary napkin 20 in a panty so that the adhesive (or other fastener) 44 contacts the inside surface of the crotch region of the panty and maintains the sanitary napkin in position within the panty during use. The side wrapping elements 50 automatically wrap around the sides of the wearer's panties by the simple action of the wearer pulling up her panties. When the panties are pulled into contact with the wearer's body, the main body portion assumes an upwardly arched configuration along the longitudinal centerline as shown in FIG. 5. The arching of the main body portion places tension on the distal edges 54 of the side wrapping elements 50. This causes the side wrapping elements 50 to fold under the panties since the side wrapping elements will tend to assume a configuration that is closest to a straight line (between the ends of the arching main body portion) when they are under tension.

The operation of the side wrapping elements 50 is distinguishable in several respects from that of conventional side flaps. Placing a sanitary napkin having conventional flaps in a pair of panties and pulling up the panties will not consistently provide the automatic sustained wraparound feature of the present invention. There are several reasons for this. Conventional flaps are not pre-disposed to wrap around the edges of a wearer's panties so they will tend to either crumple when compressed by the wearer's thighs, or simply lay against the wearer's thighs. Conventional flaps also do not retain a high degree of their fold, so that in cases where conventional flaps do wrap around the panties, they will not consistently stay wrapped. In addition, conventionally-sized flaps will have excess flap material that hangs down underneath the panties during wear. This material can move around excessively underneath the panties. The side wrapping elements of the present invention, on the other hand, have a span that is ideally just wide enough to wrap around the elastic-containing edges of the panties, but no wider, avoiding the problems associated with excess flap material.

Various alternative embodiments of the present invention are possible. For example, in alternative embodiments, the side wrapping elements 50 may be integral portions of one or more components of the main body portion. In such a case the distal end portions can be folded back and secured to the garment-facing side of the main body portion. In addition, while the side wrapping elements 50 are shown as extending from each longitudinal edge of the main body portion, there may only be one side wrapping element extending from one of the edges of the main body portion. Further, the side wrapping elements are preferably mirror images of each other, and are symmetrical about the longitudinal centerline. However, it should be understood that the shape and location of the side wrapping elements described herein are those of a preferred embodiment, and other embodiments are also possible. For instance, the side wrapping elements 50 may be offset along the longitudinal centerline more towards one end edge of the main body portion than the other.

The present invention is also applicable to other types of absorbent articles worn in the crotch region of an undergarment such as pantiliners and incontinenct articles. The terms "panty liner" or "pantiliner" refer to absorbent articles that are less bulky than sanitary napkins which are generally worn by women between their menstrual periods. Examples of suitable absorbent articles in the form of pantiliners that can be provided with the side wrapping elements described herein are disclosed in U.S. Patent 4,738,676 entitled "Pantiliner" issued to Osborn on April 19, 1988.

The term "incontinence article" refers to pads, undergarments (pads held in place by a suspension system of same type, such as a belt, or the like), inserts for absorbent articles, capacity boosters for absorbent articles, briefs, bed pads, and the like, regardless of whether they are worn by adults or other incontinent persons. Examples of suitable incontinence articles that can be provided with the side wrapping elements described herein are disclosed in U.S. Patent 5,300,054 issued to Feist, et al. on April 5, 1994 and U.S. Patent 5,304,161 issued to Noel, et al. April 19, 1994.

## Claims

1. An absorbent article (20) having a longitudinal dimension extending in a longitudinal direction (L) and a transverse dimension extending in a transverse direction (T), said absorbent article (20) comprising:
a main body portion (21) comprising an absorbent core (42), said main body portion (21) having a body-facing side (20A), a garment-facing side (20B), and a pair of longitudinal side edges (22); and
a pair of side wrapping elements (50) joined to said main body portion (21), said side wrapping elements (50) each having a transverse centerline comprising a proximal edge (52), a distal edge (54), a pair of ends (56), and a pair of distal end portions (58), said side wrapping elements (50) extending laterally outward beyond the longitudinal side edges (22) of said main body portion (21) from their proximal edges (52) to their distal edges (54), the distal end portions (58) of at least one of said side wrapping elements (50) are folded back inward toward the longitudinal centerline (L) of the absorbent article (20) and characterized in that only said distal end portions (58) are secured to the garment-facing side (20B) of the main body portion (21), and further said folding and said securing having been provided prior to use of said napkin.

2. The absorbent article (20) of Claim 1 wherien the distal end portions (58) of said at least one side wrapping element (50) are in a state of tension when they are secured to the garment-facing side (20B) of the main body portion (21).

3. The absorbent article (20) of Claim 2 wherein said distal end portions (58) are maintained in tension in the longitudinal direction (L).

4. The absorbent article (20) of Claim 2 wherein said distal end portions (58) are maintained in tension in the transverse direction (T).

5. The absorbent article (20) of Claim 1 wherein said at least one side wrapping element (50) is pre-disposed to form an acute angle relative to the garment-facing side (20B) of said main body portion (21) at the transverse centerline of said side wrapping element (50).

6. The absorbent article (20) of Claim 1 wherein said at least one side wrapping element (50) is pre-disposed to form an obtuse angle relative to the garment-facing side (20B) of said main body portion (21) at the transverse centerline of said side wrapping element (50).

7. The absorbent arrticle (20) of Claim 1 wherein said side wrapping elements (50) are extensible.

8. The absorbent arrticle (20) of Claim 7 wherein said side wrapping elements (50) are extensible in the longitudinal direction (L).

9. The absorbent arrticle (20) of Claim 7 wherein said side wrapping elements (50) are extensible in the transverse direction (T).

10. The absorbent arrticle (20) of Claim 7 wherein said side wrapping elements (50) are extensible in multiple directions.

11. The absorbent article (20) of Claim 1 wherein said main body portion (21) is extensible.

12. The absorbent article (20) of Claim 11 wherein said main body portion (21) is extensible in the longitudinal direction (L).

13. The absorbent article (20) of Claim 11 wherein said main body portion (21) is extensible in the transverse direction (T).

14. The absorbent article (20) of Claim 1 wherein said main body portion (21) is extensible in multiple directions.

## Patentansprüche

1. Ein absorbierender Artikel (20) mit einer Längsdimension, welche sich in einer Längsrichtung (L) erstreckt, und einer Querdimension, welche sich in einer Querrichtung (T) erstreckt, wobei der genannte absorbierende Artikel (20) umfaßt:
einen Hauptkörperabschnitt (21), welcher einen absorbierenden Kern (42) umfaßt, wobei der genannte Hauptkörperabschnitt (21) eine dem Körper zugewandte Seite (20A), eine der Kleidung zugewandte Seite (20B) und ein Paar Längsseitenränder (22) aufweist; und
ein Paar seitenumhüllende Elemente (50), welche mit dem genannten Hauptkörperabschnitt (21) verbunden sind, wobei die genannten seitenumhüllenden Elemente (50), jeweils mit einer Quermittellinie, einen proximalen Rand (52), einen distalen Rand (54), ein Paar Enden (56) und ein Paar von distalen Endabschnitten (58) umfassen, die genannten seitenumhüllenden Elemente (50) sich von ihren proximalen Rändern (52) zu ihren distalen Rändern (54) quer auswärts über die Längsseitenränder (22) des genannten Hauptkörperabschnitts (21) hinaus erstrecken, wobei die distalen Endabschnitte (58) von mindestens einem der genannten seitenumhüllenden Elemente (50) einwärts zurück gegen die Längsmittellinie (L) des absorbierenden Artikels (20) gefaltet sind, und dadurch gekennzeichnet ist, daß lediglich die genannten distalen Endabschnitte (58) an der der Kleidung zugewandten Seite (20B) des Hauptkörperabschnitts (21) fixiert sind und daß weiters das genannte Falten und das genannte Fixieren vor Gebrauch der genannten Vorlage beigestellt worden sind.

2. Der absorbierende Artikel (20) nach Anspruch 1, bei welchem die distalen Endabschnitte (58) des genannten mindestens einen seitenumhüllenden Elements (50) in einem unter Zugspannung gesetzten Zustand sind, wenn sie an der der Kleidung zugewandten Seite (20B) des Hauptkörperabschnitts (21) fixiert sind.

3. Der absorbierende Artikel (20) nach Anspruch 2, bei welchem die genannten distalen Endabschnitte (58) in der Längsrichtung (L) unter Zugspannung gehalten sind.

4. Der absorbierende Artikel (20) nach Anspruch 2, bei welchem die genannten distalen Endabschnitte (58) in der Querrichtung (T) unter Zugspannung gehalten sind.

5. Der absorbierende Artikel (20) nach Anspruch 1, bei welchem das genannte mindestens eine seitenumhüllende Element (50) vordisponiert ist, um einen spitzen Winkel in Bezug auf die der Kleidung zugewandte Seite (20B) des genannten Hauptkörperabschnitts (21) an der Quermittellinie des genannten seitenumhüllenden Elements (50) zu bilden.

6. Der absorbierende Artikel (20) nach Anspruch 1, bei welchem das genannte mindestens eine seitenumhüllende Element (50) vordisponiert ist, um einen stumpfen Winkel in Bezug auf die der Kleidung zugewandte Seite (20B) des genannten Hauptkörperabschnitts (21) an der Quermittellinie des genannten seitenumhüllenden Elements (50) zu bilden.

7. Der absorbierende Artikel (20) nach Anspruch 1, bei welchem die genannten seitenumhüllenden Elemente (50) verlängerbar sind.

8. Der absorbierende Artikel (20) nach Anspruch 7, bei welchem die genannten seitenumhüllenden Elemente (50) in der Längsrichtung (L) verlängerbar sind.

9. Der absorbierende Artikel (20) nach Anspruch 7, bei welchem die genannten seitenumhüllenden Elemente (50) in der Querrichtung (T) verlängerbar sind.

10. Der absorbierende Artikel (20) nach Anspruch 7, bei welchem die genannten seitenumhüllenden Elemente (50) in mehreren Richtungen verlängerbar sind.

11. Der absorbierende Artikel (20) nach Anspruch 1, bei welchem der genannte Hauptkörperabschnitt (21) verlängerbar ist.

12. Der absorbierende Artikel (20) nach Anspruch 11, bei welchem der genannte Hauptkörperabschnitt (21) in der Längsrichtung (L) verlängerbar ist.

13. Der absorbierende Artikel (20) nach Anspruch 11, bei welchem der genannte Hauptkörperabschnitt (21) in der Querrichtung (T) verlängerbar ist.

14. Der absorbierende Artikel (20) nach Anspruch 1, bei welchem der genannte Hauptkörperabschnitt (21) in mehreren Richtungen verlängerbar ist.

## Revendications

1. Article absorbant (20) ayant une dimension longitudinale s'étendant dans une direction longitudinale (L) et une dimension transversale s'étendant dans une direction transversale (T), ledit article absorbant (20) comprenant :
une partie de corps principal (21) comprenant une âme absorbante (42), ladite partie de corps principal (21) présentant un côté faisant face au corps (20A), un côté faisant face au vêtement (20B), et une paire de bords latéraux longitudinaux (22); et
une paire d'éléments enveloppants latéraux (50) réunis à ladite partie de corps principal (21), lesdits éléments enveloppants latéraux (50) présentant chacun une ligne médiane transversale comprenant un bord proximal (52), un bord distal (54), une paire d'extrémités (56), et une paire de parties d'extrémités distales (58), lesdits éléments enveloppants latéraux (50) s'étendant latéralement vers l'extérieur au-delà des bords latéraux longitudinaux (22) de ladite partie de corps principal (21), de leurs bords proximaux (52) à leurs bords distaux (54), les parties d'extrémités distales (58) d'au moins un desdits éléments enveloppants latéraux (50) sont repliées vers l'intérieur en direction de la ligne médiane longitudinale (L) de l'article absorbant (20), et caractérisé en ce que seules lesdites parties d'extrémités distales (58) sont assujetties au côté faisant face au vêtement (20B) de la partie de corps principal (21) et, en outre, ledit pliage et ledit assujettissement ayant été prévus avant d'utiliser ladite serviette.

2. Article absorbant (20) selon la revendication 1, dans lequel les parties d'extrémités distales (58) au moins dudit élément enveloppant latéral (50) sont dans un état de tension quand elles sont assujetties au côté faisant face au vêtement (20B) de la partie de corps principal (21).

3. Article absorbant (20) selon la revendication 2, dans lequel lesdites parties d'extrémités distales (58) sont maintenues en tension dans la direction longitudinale (L).

4. Article absorbant (20) selon la revendication 2, dans lequel lesdites parties d'extrémités distales (58) sont maintenues en tension dans la direction transversale (T).

5. Article absorbant (20) selon la revendication 1, dans lequel au moins ledit élément enveloppant latéral (50) est prédisposé pour former un angle aigu par rapport au côté faisant face au vêtement (20B) de ladite partie de corps principal (21) au niveau de la ligne médiane transversale dudit élément enveloppant latéral (50).

6. Article absorbant (20) selon la revendication 1, dans lequel au moins ledit élément enveloppant latéral (50) est prédisposé pour former un angle obtus par rapport au côté faisant face au vêtement (20B) de ladite partie de corps principal (21) au niveau de la ligne médiane transversale dudit élément enveloppant latéral (50).

7. Article absorbant (20) selon la revendication 1, dans lequel lesdits éléments enveloppants latéraux (50) sont extensibles.

8. Article absorbant (20) selon la revendication 7, dans lequel lesdits éléments enveloppants latéraux (50) sont extensibles dans la direction longitudinale (L).

9. Article absorbant (20) selon la revendication 7, dans lequel lesdits éléments enveloppants latéraux (50) sont extensibles dans la direction transversale (T).

10. Article absorbant (20) selon la revendication 7, dans lequel lesdits éléments enveloppants latéraux (50) sont extensibles dans de multiples directions.

11. Article absorbant (20) selon la revendication 1, dans lequel ladite partie de corps principal (21) est extensible.

12. Article absorbant (20) selon la revendication 11, dans lequel ladite partie de corps principal (21) est extensible dans la direction longitudinale (L).

13. Article absorbant (20) selon la revendication 11, dans lequel ladite partie de corps principal (21) est extensible dans la direction transversale (T).

14. Article absorbant (20) selon la revendication 1, dans lequel ladite partie de corps principal (21) est extensible dans de multiples directions.
